# EUROPEAN PATENT APPLICATION

(11) **EP 2 283 885 A1**
(43) Date of publication of application: **16.02.2011**
(21) Application number: 09010323.5
(22) Date of filing: 11.08.2009
(51) Int. Cl.: A61M 5/19, A61M 5/24

(54) **Dosing unit for an injection device**

(71) Applicant: Sanofi-Aventis Deutschland GmbH, 65929 Frankfurt am Main (DE)
(72) Inventor: The designation of the inventor has not yet been filed

(57) **Abstract**

A dosing unit for an injection device comprising a dosing chamber (102) and a first flow channel (104) and a second flow channel (106), the first and second flow channels each having a distal end and a proximal end. The proximal end of the first flow channel is in fluid engagement with a first needle (116) and the distal end of the first flow channel is in fluid engagement with the dosing chamber (102). The proximal end of the second flow channel is in fluid engagement with a second needle (118) and the distal end of the second flow channel is in fluid engagement with the dosing chamber (102). During a first dose setting operation, the first needle (116) is in fluid communication with a first cartridge containing a first medicament, the second needle (118) is in fluid communication with a second cartridge containing a second medicament, and at least one of the first and second medicament is transferred from at least one of the first or second cartridges through at least one of the first and second flow channels to the dosing chamber (102).

## Description

### Field of Application

The present patent application relates to a dosing unit for an injection device, and more particularly to a dosing unit connectable to two or more medicament reservoirs to form a dosing system. Furthermore, the application relates to an injection device and to a method for variable dosing of a plurality of medicaments in an injection device.

### Background

The art has recognized a need to inject two or more medications simultaneously and has disclosed injection devices that hold two cartridges of medication. For example, it is medically desirable in the treatment of certain conditions of the human body to administer several types and dosage amounts of medication simultaneously. The treatment of diabetes is one such example. Different types of insulin act in different fashions. A fast acting insulin works quickly but for a short period of time after administration, whereas a longer acting insulin takes effect more slowly, but for a longer period of time.

The art has disclosed medical devices in which two reservoirs or cartridges, each containing a suitable component (*i*.*e*., medicament), are integrated as active substance reservoirs. Each reservoir or cartridge is provided with its own injection needle, and the two components are applied separately from one another.

The art has also disclosed a medical device including the combination of two cartridges in the device. Such medical devices may apply a pre-defined ratio via two cartridges, each cartridge containing an active substance formulation, with a connection part to the injection needle. The two components are combined by being brought together in a common injection needle.

Problems are associated with such devices discussed above, however. In devices having two needles, the active substance solutions cannot be mixed together before application. Further, the need to insert two needles may cause patients both physical and psychological problems.

When several components are brought together in one common injection needle, there is a possibility of the volumetric flows passing partially into the delivery channels of the other dosed components. Therefore, such devices entail the risk of back-contamination by the respective other component.

Accordingly, there exists a need to provide users of such injection devices with an easy-to-use device that allows for the simultaneous injection of two or more medicaments. The disclosed dosing unit for injection devices solves the above-described problems by providing a dosing unit including a dosing chamber, a first flow channel, and a second flow channel. These and other advantages will become evident from the following more detailed description of the invention.

### SUMMARY

According to an exemplary arrangement, a dosing unit is disclosed comprising a dosing chamber and a first flow channel and a second flow channel. The first and second flow channels each having distal and proximal ends. The proximal end of the first flow channel is in fluid engagement with a first needle and the distal end of the first flow channel is in fluid engagement with the dosing chamber. The proximal end of the second flow channel is in fluid engagement with a second needle and the distal end of the second flow channel is in fluid engagement with the dosing chamber.

According to another embodiment, during a first dose setting operation, the first needle is in fluid communication with a first cartridge which may contain a first volume of a first medicament, the second needle is in fluid communication with a second cartridge which may contain a second volume of a second medicament. At least one of the first or second medicaments is transferred from at least one of the first or second cartridges through at least one of the first and second flow channels into the dosing chamber. In an alternative arrangement, more than two medicaments may be provided in individual reservoirs.

The dosing unit may also include an injection needle operable to communicate with the dosing chamber. The dosing unit may also include a dose stopper that may be (i) connected to a piston rod. The dose stopper may be operable to move with respect to the dosing chamber, independent of its connection with a piston rod. Preferably, the stopper may be (ii) operable to move axially with respect to the dosing chamber. During injection, displacement of the dose stopper displaces a medicament contained in the dosing chamber in the direction of the injection needle. Preferably, the piston rod is subjected to forward movement, and the forward movement of the piston rod displaces the medicament in the dosing chamber in the direction of the injection needle. In accordance with an embodiment, during a dose setting operation, the first and second medicaments are mixed together. Therefore, a dosing unit in accordance with an embodiment may allow for variable or pre-set dosing of a plurality of medicaments through a common injection needle.

These as well as other advantages of various aspects of the present invention will become apparent to those of ordinary skill in the art by reading the following detailed description, with appropriate reference to the accompanying drawings. The scope of the invention is defined by the content of the claims. The invention is not limited to specific embodiments but comprises any combination of elements of different embodiments. Moreover, the invention comprises any combination of claims and any combination of features disclosed by the claims.

### BRIEF DESCRIPTION OF THE DRAWINGS

Exemplary embodiments are described herein with reference to the drawings, in which:
Figure 1 illustrates a first arrangement of a dosing unit;
Figure 2a illustrates an exploded cross-sectional view of the dosing unit illustrated in Figure 1;
Figure 2b illustrates an exploded perspective view of the dosing unit illustrated in Figure 1;
Figure 3 illustrates a dosing system comprising an injection device and a dosing unit, such as the dosing unit illustrated in Figure 1;
Figures 4a, 4b, and 4c illustrate respective arrangements of a valve system for use with a dosing unit, such as the dosing unit illustrated in Figure 1;
Figures 5a and 5b illustrate respective arrangements of an injection-needle access mechanism for use with a dosing unit, such as the dosing unit illustrated in Figure 1;
Figure 6 illustrates a second arrangement of a dosing unit;
Figure 7 illustrates a third arrangement of a dosing unit; and
Figure 8 illustrates a side view of the removable needle-shield illustrated in
Figure 7.

### DETAILED DESCRIPTION

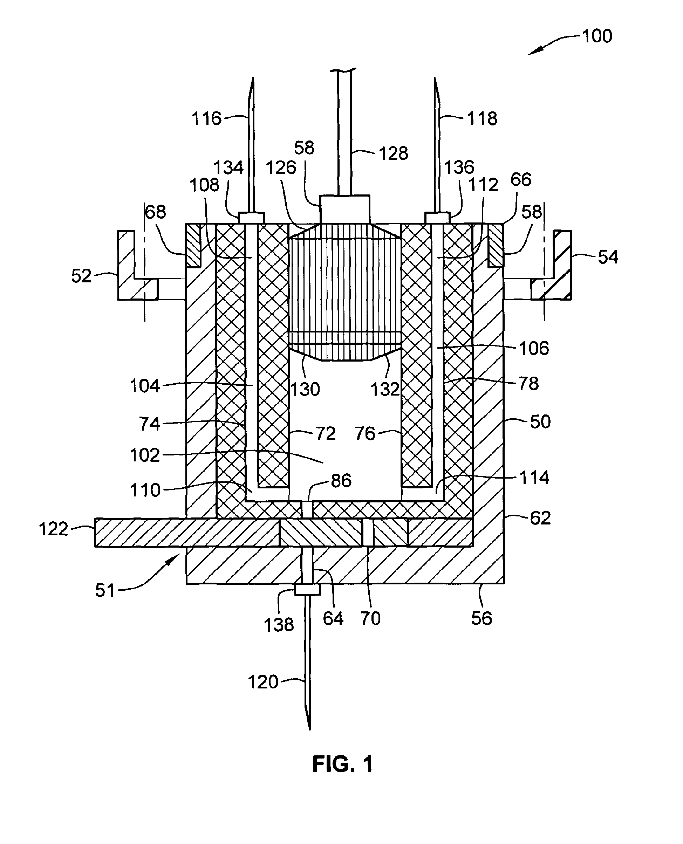

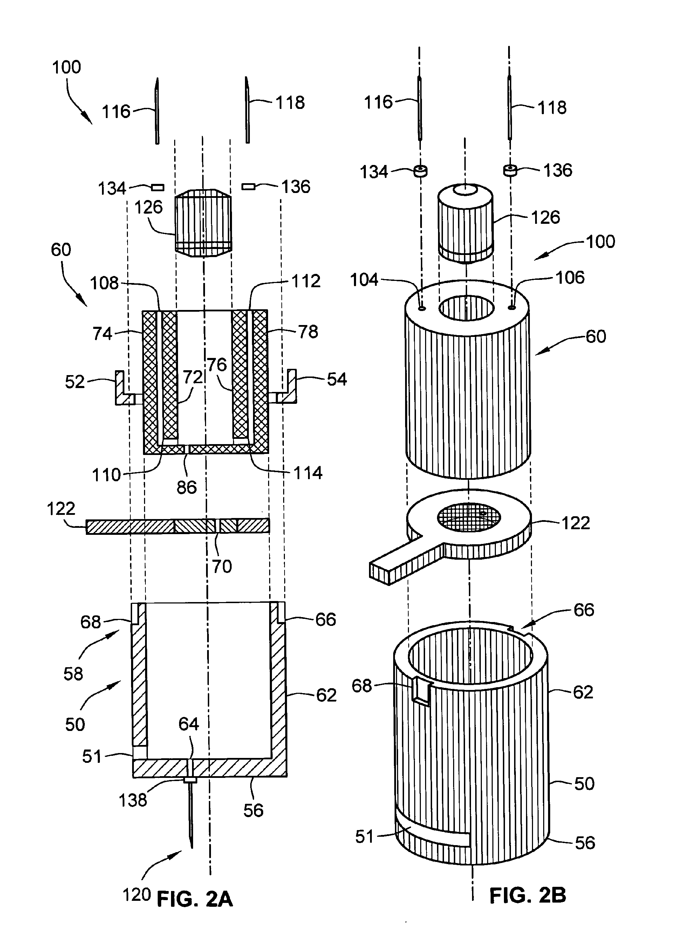

Figure 1 illustrates a first arrangement of a dosing unit. Figure 2a illustrates an exploded cross-sectional view of the dosing unit illustrated in Figure 1 and Figure 2b illustrates an exploded perspective view of the dosing unit illustrated in Figure 1. Referring now to Figures 1-2, such dosing unit allows a plurality of individual medicaments to be dosed in a variable or pre-set manner. In an exemplary preferred arrangement, the dosing unit 100 allows for the plurality of individual medicaments to be dosed in a variable manner or a pre-set manner free from back contamination. In the exemplary dosing unit 100, two medicaments may be brought together and mixed together in a dosing chamber 102. In an alternative exemplary dosing unit, three, four, five or more medicaments may be brought together and mixed together in a dosing chamber. However, for the sake of clarity in describing the illustrated arrangements, only two medicament reservoirs are illustrated. However, as those of ordinary skill in the art will recognize, more than two medicaments may be utilized with the various disclosed dosing unit arrangements.

These mixed medicaments can then be administered (*i*.*e*., by way of a single injection) by a user to a single injection site. This can be accomplished by using an injection system comprising a dosing unit 100 and an injection device, such as a pen type injection device. One advantage of the dosing chamber 102 is that it permits a generally high degree of dosing accuracy and mixing of at least two medicaments before administration.

Dosing unit 100 comprises a main housing 50 that can be generally circular in shape and that houses a cylindrical member 60. Preferably, the main housing 50 defines an access path 64 and the cylindrical member 60 defines a cylindrical member access path 86. This main housing 50 also preferably includes at least a first and a second coupling member 52, 54. Coupling members 52, 54 allow for the dosing unit 100 to be either releasably or non-releasably coupled to an injection device.

The main housing 50 further provides a housing means for the generally cylindrical member 60. This cylindrical member 60 defines at least a first flow channel 104 and a second flow channel 106. Those of skill in the art will recognize that the generally cylindrical member 60 could define other flow channels as well. Alternatively, or in addition, an outer wall 62 of the main housing 50 may comprise a coupling mechanism 66, 68 for releasably connecting the main housing 50 to an injection device.

The generally cylindrical member 60 defines a first flow channel 104 and a second flow channel 106. Although Figures 1 and 2a-b only show two flow channels, the dosing unit 100 may include more than a first and a second flow channels 104, 106. First flow channel 104 extends from a distal end 110 near a distal end 56 of the main housing 50 and a proximal end 108 near a proximal end of the main housing 50. Preferably, the first flow channel 104 is defined between a first flow channel inner wall 72 and a first flow channel outer wall 74. Similarly, second flow channel 106 extends from a distal end 114 to a proximal end 112 and is defined between a second flow channel inner wall 76 and a second flow channel outer wall 78.

Proximal end 108 of the first flow channel 104 is in fluid engagement with a first needle 116, and proximal end 112 of the second flow channel 106 is in fluid engagement with a second needle 118. In one arrangement, sealing rings, such as sealing rings 134 and 136, may be provided so as to provide a fluid seal between the first and second flow channels 104, 106 and the first and second needles 116, 118, respectively.

Distal end 110 of the first flow channel 104 and distal end 114 of the second flow channel 106 are each in fluid communication with the dosing chamber 102. As illustrated, the flow channels 104, 106 are linear flow channels. However, alternatively shaped flow channels may also be used. These flow channels provide a path that flows axially from the needle and then into the dosing chamber 102. Accordingly, a first medicament that flows through flow channel 104 may flow into the dosing chamber 102 and then subsequently a second medicament that flows through channel 106 may flow into the dosing chamber 102. It should be understood that other types of flow paths from the needle to the dosing chamber are possible as well. As just one example, the flow channel may have a path that flows from the needle to the dosing chamber at a 30 degree angle.

Dosing unit 100 may also include a third injection needle 120 that is operable when in fluid communication with the dosing chamber 102. There may be a sealing ring, such as sealing ring 138, located around the third injection needle 120 in order to seal the connection between the injection needle 120 and access to the dosing chamber 102.

Preferably, during a dose setting step, the third injection needle 120 is not in fluid engagement with the dosing chamber 102. This lack of fluid engagement is illustrated in Figure 1. In other words, when a user selects an amount of medication from one of the cartridges attached to the injection device and provides this set dose to the dosing chamber 102, the third injection needle 120 is not in fluid engagement with the dosing chamber 102. This lack of access allows for the mixing of a first and second medicament in the dosing chamber 102 prior to an injection step of injecting this medicament mixture via the third injection needle 120. Further, this lack of access allows for filling the dosing chamber 102 with the desired volume of medicament(s) prior to injection via the injection needle 120.

In order to achieve this lack of access to the injection needle 120 during dosing, the dosing unit 100 comprises an injection needle access mechanism 122. Injection needle access mechanism 122 preferably accomplishes a number of things. First, after some type of manipulation of the access mechanism 122, this mechanism 122 prevents access from the dosing chamber 102 to the third injection needle 120 during a dose setting step. In addition, again after some type of manipulation, this mechanism 122 allows access from the dosing chamber 102 to the injection needle 120 during injection.

As illustrated in Figure 1, the injection-needle access mechanism 122 is removably disposed between the dosing chamber 102 and the injection needle 120. More specifically, the injection-needle access mechanism 122 is removably disposed between the generally cylindrical member 60 and the main housing 50. The injection needle access mechanism 122 may be removably inserted into a slot 51 defined at the distal end 56 of the main housing 50.

In one arrangement, the injection-needle access mechanism 122 comprises a generally flat member and this generally flat member defines at least one access path 70. As illustrated in Figure 1, in one orientation, the injection-needle access mechanism 122 blocks a dose injection path from dosing chamber 106 to injection needle 120 because the injection needle access mechanism access path 70 is not in alignment with the cylindrical member access path 86 and the main housing access path 64 defined by the main housing 50. Once a user manipulates the access mechanism 122 so that these paths 86 and 64 are in alignment, the dosing unit 100 will be in a dose injection ready state.

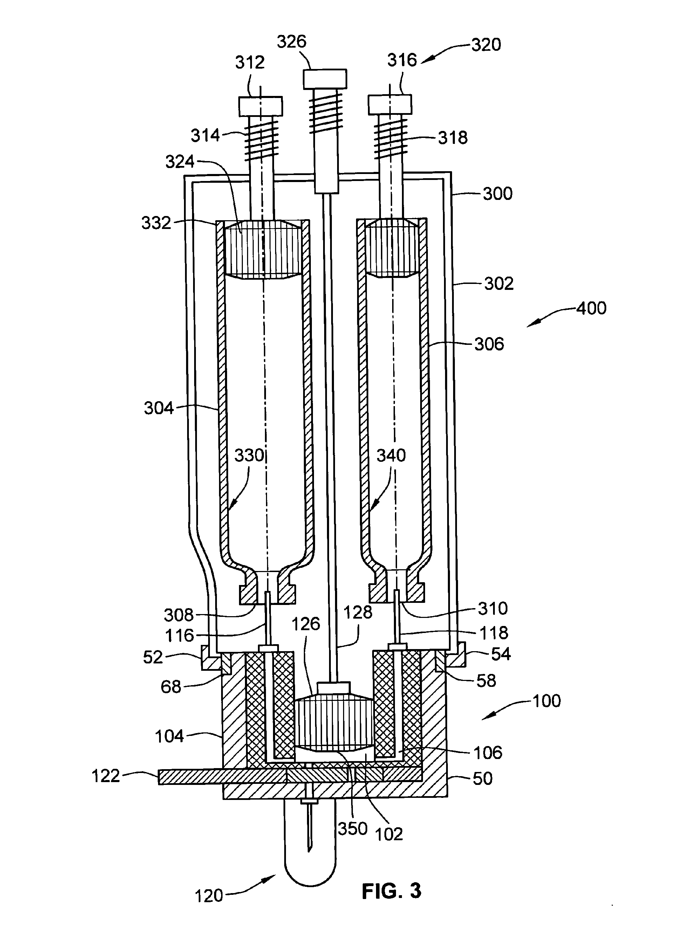

Figure 3 illustrates a dosing system 400 comprising a dosing unit 100 and an injection device 300. In one arrangement, the dosing unit 100 is releasably connected to the injection device 300. Preferably, such an injection device 300 comprises a pen type injection device. The injection device 300 may include a generally tubular housing 302 and a dose setting mechanism 320 comprising a number of dose setting knobs 312, 316, and 326.

In this preferred arrangement, the generally tubular housing houses a first reservoir or cartridge 304 and a second reservoir or cartridge 306. The first and second cartridges 304, 306 may comprise conventional cartridges comprising a glass enclosure containing a moveable bung at one end and a resealable membrane at another end. For example, first cartridge 304 comprises a 3.0 milli-liter (ml) glass enclosure 322 containing a moveable bung 324 at a proximal end and a resealable membrane 308 at a distal end.

In one preferred arrangement, the cartridges 304, 306 may comprise cartridges having varying volumes. For example, as illustrated, the first cartridge 304 comprises 3.0 ml of a first medication 330 and the second cartridge 306 comprises 1.7 ml of a second medication 340. One reason for this difference in cartridge size is that, during a dose setting step wherein a user sets a dose of medication contained within either cartridge 304, 306, a specific injection may require a different size dose of the first medication 330 than the size dose of the second medication 340. As just one example, the first cartridge 304 may contain a long acting insulin as the first medication 330 and the second cartridge 306 may contain a short acting insulin or alternatively may contain a GLP-1 as the second medication 340. In such an injection system, the user would select a dose from the long acting insulin that is different than the dose selected from the short acting insulin or from the GLP-1. Alternatively, the user would select a pre-set dose (*i*.*e*., a non-variable dose) from either the first or the second medicament.

The dosing unit 100 may be connected to the device 300 by means of outer connecting members 52, 54 (e.g., a connecting nut) and inner connecting members 58 consisting of centering devices 66 and 68 (e.g., channel). Other examples for outer and inner connection members are possible as well. The inner and outer connecting members preferably secure connection of the dosing unit 100 to the injection device 300. For example, the dosing unit 100 may be mechanically connected to the housing 302 using an appropriate connection part. The connection part may include, for example, an axial connection and/or a snap lock or snap fit connection. Other examples of axial connecting members are possible as well.

When the dosing unit 100 is connected to the injection device 300, the first needle 116 of the dosing unit 100 pierces a resealable membrane 308 of the first cartridge 304. As such, the first needle 116 will now be in fluid engagement with the first medicament 330 contained in the first cartridge 304. Similarly, once the second needle 118 of the dosing unit 100 pierces a resealable membrane 310 of the second cartridge 306, this needle 118 will then be in fluid engagement with the second medicament 340 contained in the second cartridge 306. The act of attaching the dosing unit 100 to the injection device 300 may connect the respective needles 116, 118 to the cartridges 304, 306, respectively.

When dosing unit 100 is connected to injection device 300, a user may use the injection system 400 to inject at least one of the first and second medicaments, 330 and 340. For example, the user may set doses of the two types of medicament 330, 340 at the same time. Further, it is also possible that a user may administer these two different medicaments independently with the same injection device. For example, a user could alternate between injecting the first medicament and the second medicament. Such use may prove beneficial for administering both long-acting insulin and short-acting insulin with the same injection device.

During a dose selection step, a user selects an appropriate dose of medication. In an exemplary embodiment, a user may select to mix a certain amount of the first medicament 330 and a certain amount of the second medicament 340. A user may select a certain amount of the first medicament 330 for the dose. The user may then begin to dose this amount, and this amount of the first medicament 330 may flow from the first reservoir 304 to the dosing chamber 102. Specifically, injection device 300 may include a dosing knob, such as dosing knob 312, which may be connected to a first piston rod, such as first piston rod 314. When a user selects a certain amount of the first medicament 330, the first piston rod 314 may move the dosing knob 312 distally. This downward movement of piston rod 314 acts on the moveable bung 324 and therefore pushes the first medicament 330 from the first cartridge 304 through the first needle 116 and into the flow channel 104. Similarly, a second dosing knob 316 may be connected to a second piston rod 318. When a user selects a certain amount of the second medicament 340, the second piston rod 318 moves the second dosing knob 316 also distally. This downward movement acts on the bung contained within the second cartridge 306 and pushes the second medicament 340 through the second needle 118 into the second flow channel 106. The selected amount of the first medication 330 and the selected amount of the second medication 340 may then be mixed in dosing chamber 106 prior to injection to create a mixed medication 350.

Preferably, the dosing unit 100 includes a dosing stopper 126. In one arrangement, the dosing unit 100 comprises the dosing stopper 126. Alternatively, the dosing stopper 126 may comprise a separate independent component or alternatively may comprise a part of the injection device. The dosing stopper 126 may be connected to a piston rod, such as piston rod 128. The dosing stopper 126 is preferably operable to move axially with respect to the dosing chamber 102. Preferably, prior to dosing, the dosing stopper 126 is located substantially near the bottom of the dosing chamber 102. When either the first or the second medicament 330, 340 flows from either the first flow channel 104 or the second flow channel 106, respectively, the flow of the liquid may force the dosing stopper 126 to move proximally or upward.

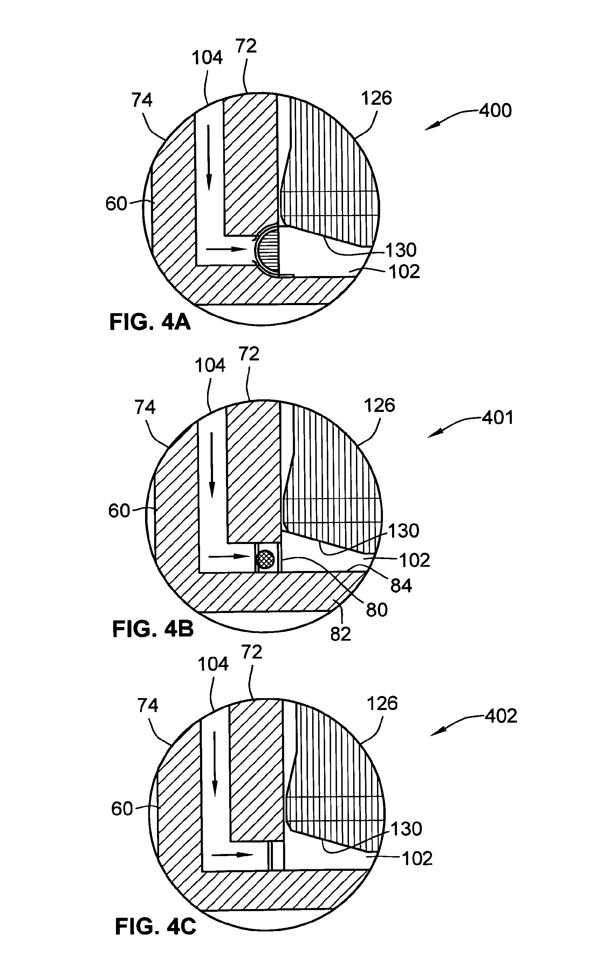

In an exemplary arrangement, the dosing stopper 126 has at least one beveled edge. In the illustrated dosing stopper 126 of Figure 1, this dosing stopper 126 comprises a plurality of beveled edges, beveled edges 130 and 132. These beveled edges 130, 132 are clearly illustrated in Figure 1. The dosing stopper edges 130 and 132 may beveled in such a way that medicament flowing in radially to dosing chamber 102 so as to deploy forces in the axial direction and lifts the dosing stopper 126 so that the dosing stopper 126 moves in a proximal direction. This action is depicted in Figures 4a, 4b, and 4c.

Alternatively, the dosing stopper 126 may be provided on the end faces with a drainage channel or channels or with knobs in order to allow an active substance solution to flow under. This tends to introduce a force to lift the stopper from a distal position. Essentially, this reduces any potential dead space that may reside in the dosing chamber 102.

In accordance with a preferred arrangement, back contamination of a mixed medicament 350 comprising a mixture of the first medication 330 and the second medication 340 into the first and second flow channels 104, 106 from the dosing chamber 102 may be avoided with dosing unit 100. Beneficially, avoiding back-contamination prevents an undesired mixing of different types of medicaments in the separate flow channels 104, 106.

In one arrangement, such back contamination may be avoided by setting suitable pressure conditions or by implementing a valve system within the dosing unit. In a pressure-regulated system, the pressure of the first medicament and the second medicament may be equal during filling of the dosing chamber 102 in order to avoid back contamination.

In the case of unequal pressure conditions, which may correspond to unequal volumetric flows, valve systems may preferably be used in Applicants' injection system, such as the system 400 illustrated in Figure 3. In one preferred arrangement, the counter-pressure of the dosing stopper 126 is preferably smaller than that of the first medicament and the second medicament in order to shift the dosing stopper 126 from an initial start position into a dosing or subsequent position. The dosing units of the medicaments are fixed against return movement.

Figures 4a, 4b, and 4c show examples of various non-return valve systems. Such valve system may be any type of valve system known in the art. For example, valve system may be a half O-ring-valve system 400 as depicted in Figure 4a. The half O-ring-valve system 400 preferably lies in a peripheral groove defined between the first flow channel inner wall 72 and the first flow channel outer call 74. In such an arrangement, movement of the dosing stopper 126 is not impeded. Alternatively, valve system may comprise a ball-valve system 401 as depicted in Figure 4b. Such an arrangement comprises a ball 80 positioned under or near the first flow channel inner wall 72 and a top surface 84 of a bottom portion 82 of the cylindrical member 60. Still alternatively, the valve system may comprise a membrane-valve system 402 as depicted in Figure 4c. In the first flow channel 104 a valve is placed (e.g. a diaphragm valve), that allows the flow in only one direction, i.e. from flow channel 104 to the compartment 102. With such a valve system, there is no return flow possible from the compartment 102 in first flow channel 104. Other valve systems are possible as well. Further, it should be noted that, although Figures 4a, 4b, 4c only depict a valve system disposed in the first flow channel 104, the second flow channel 106 may also have a valve system.

After the first medicament 330 and the second medicament 340 are selected and then mixed together in the dosing chamber 102 to form the mixed medication 350, access to the third injection needle 120 may be freed in order to allow for injection.

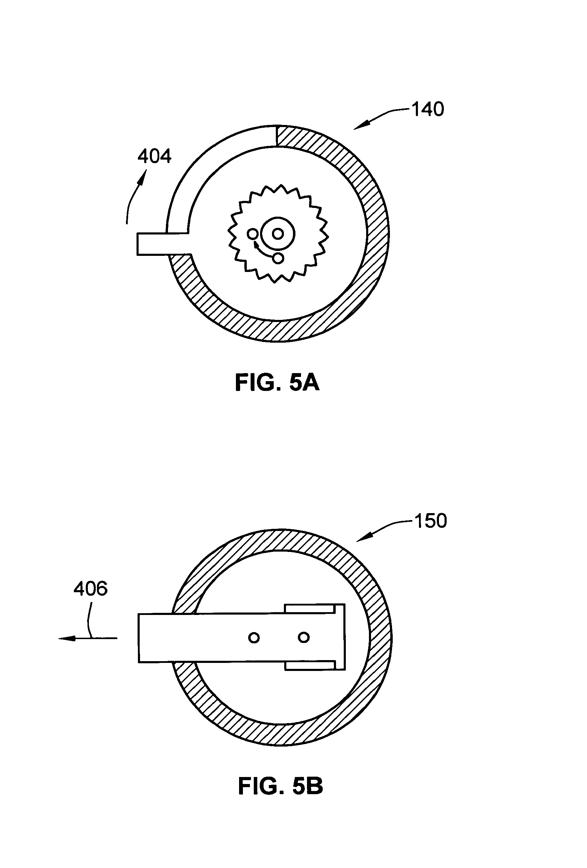

This access for an injection of a mixed medication to occur may be accomplished via movement of the injection-needle access mechanism 122 so as to align the first and the second access paths 64, 70. The movement of the injection-needle access mechanism 122 may be, for example, a rotary movement or a translational movement. Embodiments of the injection-needle access mechanism 122 are further depicted in Figures 5a and 5b. For example, Figure 5a depicts a rotary injection-needle access mechanism 140. That is, by turning the needle access mechanism 122 in the clockwise direction 404, this will align the first and the second access paths 64, 70. Figure 5b depicts a sliding injection-needle access mechanism 150. In this arrangement, movement of injection-needle access mechanism 122 in the axial direction 406 will result in aligning the first access path in the injection-needle access mechanism 122 with the second flow path defined between the dosing chamber 102 and the third injection needle 120.

After the main housing access path 64 and the injection needle access mechanism access path 70 have been aligned, a user may now inject the medicament mixture 350 located in the dosing chamber 102. In order to inject this medicament mixture 350, the user subjects the piston rod 128 to forward movement. In this manner the dosing stopper 126 displaces the volume of the dosing chamber 102 in a distal direction, in the direction of the third injection needle 120.

The third injection needle 120 may be connected to the dosing unit 100 in various ways. The connection of the injection needle 120 may be realized by performing form-fit or force-fit variants commonly used in the art. These may include, for example, screw closure, plug closure, bayonet closure, and clamp closure. Other examples are possible as well. Further, the injection needle can be fit by rotary movement or translational movement.

In one arrangement, the dosing unit 100 may be securely connected to the third injection needle 120. Preferably, such a dosing unit may be available as a complete, separate unit offered and/or sold in a sterile package. This unit may then be connected to an injection device for a single use, and then dismantled from the injection device and disposed of after the single use. One benefit of such use is that it allows for the use of a new, sterile dosing unit on the same injection device. Further, the dosing unit may be used in conjunction with a reusable (*i*.*e*., non-disposable) injection device and/or a non-reusable (*i*.*e*., disposable) injection device. Preferably, such dosing unit may be used in conjunction with a reusable (*i*.*e*., non-disposable) pen type injection device and/or a non-reusable (*i*.*e*., disposable) pen type injection device.

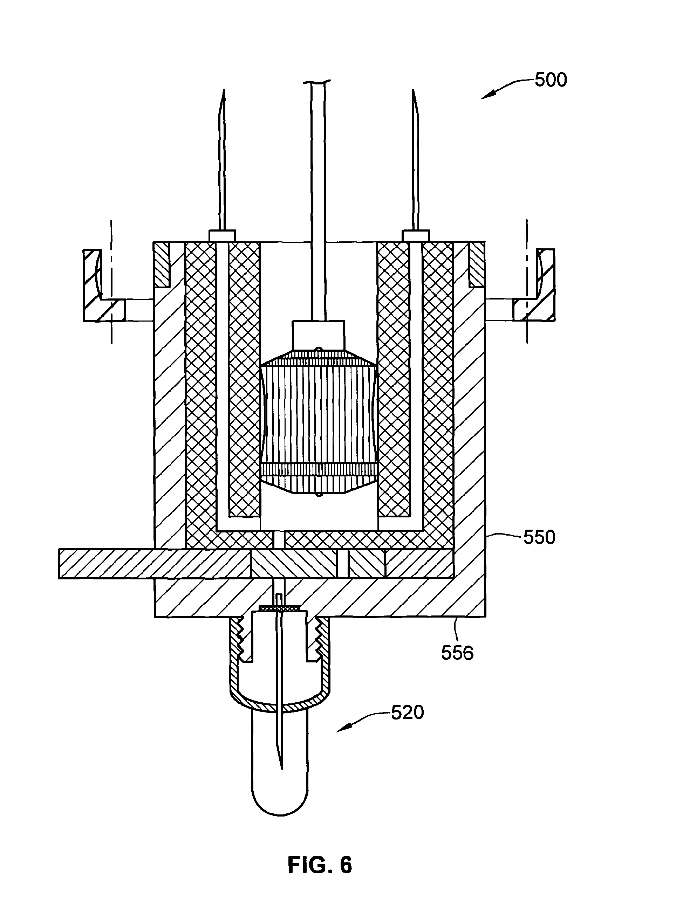

Figure 6 illustrates an alternative arrangement for a dosing unit 500. Dosing unit 500 has similar features as the dosing unit 100 illustrated in Figure 1. However, in this alternative arrangement, the dosing unit 500 may be designed as a reusable dosing unit that can be attached to an injection device (e.g., reusable or non-reusable injection device) for a series of multiple uses. In this case, the injection needle 520 and the connection of the injection needle to the dosing unit 500 may be designed so that the injection needle can be replaced after each use. As illustrated, the distal end 556 of the dosing unit main housing 550 comprises a thread so that a standard commercial injection needle provided with a threaded hub may be threaded on to the 556. One advantage of such a reusable dosing unit 500 operable with such a replaceable needle 520, is that it allows for the use of a new, sterile needle for each injection.

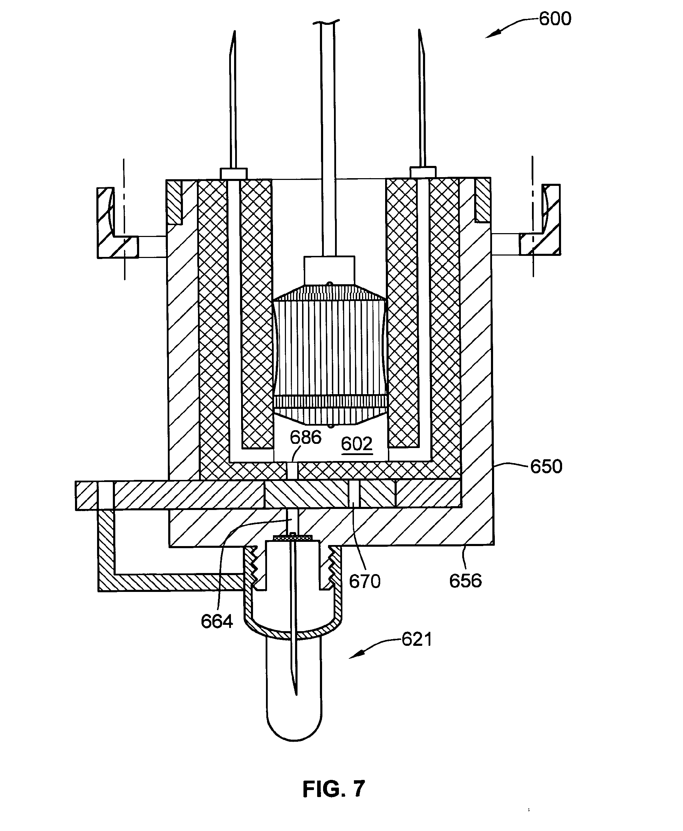

Figure 7 illustrates yet another alternative dosing unit arrangement 600. Dosing unit 600 has similar features as the dosing unit 500 illustrated in Figure 6. However, in this alternative dosing unit arrangement 600, a movable fixing connection of the injection needle-shield 621 to the needle access mechanism 122 is made. The injection needle 120 is already fixed to the dosing unit 600. In order to open and remove the needle-shield 621 one has to turn the needle-shield 621 and the connected needle access mechanism 122, e.g. in clock wise direction. By moving the needle-shield 621 the activator 122 enables connection of the dosing chamber 602 through the fluid path 686, 670, and 664 to the injection needle.

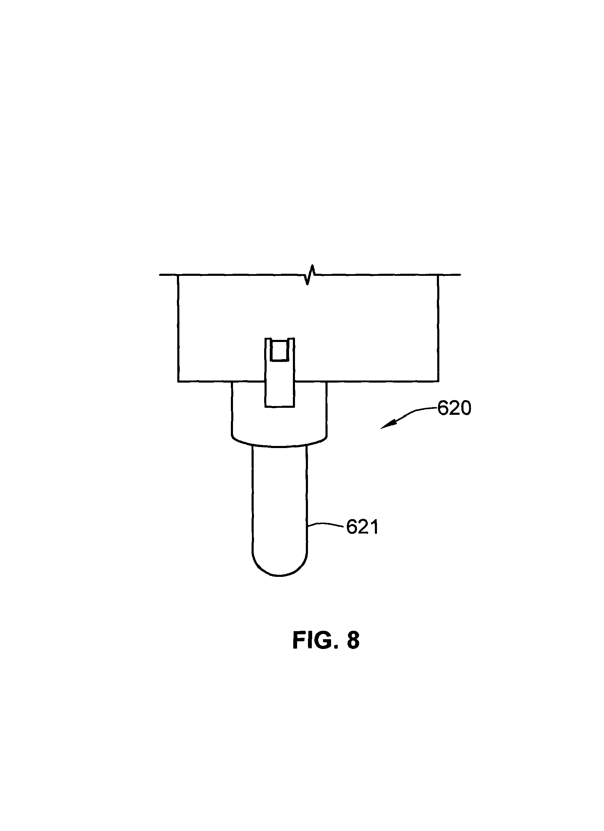

Figure 8 shows a side view of the construction of the injection needle-shield 620 and its connection to the needle access mechanism 122 as illustrated in Figure 7.

In a further embodiment, the injection needle 120 can be removably attached to the dosing unit 600 as described in the general alternatives of Fig. 6 or Fig. 7, when access to the dosing chamber 602 is prevented. In this alternative arrangement, it is advantageous that the dosing unit 600 can remain fixed to the device reservoirs 304 and 306 and only the injection needle 120 has to be exchanged for each administration of the medicament.

The various components of the dosing unit 100 may be manufactured from various manufacturing materials. For example, the manufacturing materials may include, for example, glass, plastic, and metal. Additionally or alternatively, the various components may be manufactured by a combination of these materials. For example, the main body of the dosing unit 100 may comprise glass. The attachment pieces may comprise plastics (e.g., aliphatic, cyclic or bicyclic polyolefins such as PP, PE, PET, COC and COP, or PVC). The sealing rings 134, 136, and 138 may be made of a wide variety of materials (e.g., halobutyl-rubber, isoprene- and polyisoprene rubber, EPDM, PVC). Further, the sealing rings may adhere to the surface in various ways (e.g., adhesion by conventional pharma-compatible adhesive, adhesion by solvents, adhesion by plasma-induced covalent bonding, welding, e.g. by ultrasonic welding, high-frequency welding).

Exemplary embodiments of the present invention have been described. Those skilled in the art will understand, however, that changes and modifications may be made to these embodiments without departing from the true scope and spirit of the present invention, which is defined by the claims.

### References:

- 100: dosing unit
- 102: dosing chamber
- 104: first flow channel
- 106: second flow channel
- 108: first flow channel proximal end
- 110: first flow channel distal end
- 112: second flow channel proximal end
- 114: second flow channel distal end
- 116: first needle
- 118: second needle
- 120: third injection needle
- 122: injection needle access mechanism
- 126: dosing stopper
- 128: piston rod
- 134, 136, 138: sealing rings
- 140: access mechanism
- 150: access mechanism
- 300: injection device
- 302: tubular housing
- 304: first reservoir or cartridge
- 306: second reservoir or cartridge
- 308: resealable membrane
- 312, 316, 326: dose setting knobs
- 314: first piston rod
- 318: second piston rod
- 320: setting mechanism
- 322: glass enclosure
- 324: moveable bung
- 330: first medication
- 340: second medication
- 350: mixed medication
- 400: dosing system
- 401: ball-valve system
- 402: membrane-valve system
- 404: clockwise direction
- 406: axial direction
- 50: main housing
- 51: slot
- 52: first coupling member/outer connecting member
- 54: second coupling member/ outer connecting member
- 56: distal end
- 58: inner connecting members
- 500: dosing unit
- 520: injection needle
- 550: dosing unit main housing
- 556: distal end
- 60: cylindrical member
- 62: outer wall
- 64: access path
- 66, 68: coupling mechanism/centering devices
- 600: dosing unit arrangement
- 602: dosing chamber
- 620: injection needle-shield
- 621: needle-shield
- 664, 670, 686: fluid path
- 70: access path
- 72: first flow channel inner wall
- 74: first flow channel outer wall
- 76: second flow channel inner wall
- 78: second flow channel outer wall
- 80: ball
- 82: bottom portion
- 84: top surface
- 86: cylindrical member access path

## Claims

1. A dosing unit (100) for an injection device comprising:
a dosing chamber (102);
a first flow channel (104) and a second flow channel (106), the first and second flow channels (104, 106) each having a distal end (110) and a proximal end (108),
wherein the proximal end (108) of the first flow channel (104) is in fluid engagement with a first needle (116) and
the distal end (110) of the first flow channel (104) is in fluid engagement with the dosing chamber (102),
wherein the proximal end (112) of the second flow channel (106) is in fluid engagement with a second needle (118) and
the distal end (114) of the second flow channel (106) is in fluid engagement with the dosing chamber (102).

2. The dosing unit (100) of claim 1 wherein, during a first dose setting operation,
the first needle (116) is in fluid communication with a first cartridge (304) containing a first medicament (330) ,
the second needle (118) is in fluid communication with a second cartridge (306) containing a second medicament (340), and
at least one of the first and second medicament (330, 340) is transferred from at least one of the first or second cartridges (304, 306) through at least one of the first and second flow channels (104, 106) to the dosing chamber (102).

3. The dosing unit (100) of claim 2, wherein during a subsequent dose setting operation, the first and second medicaments (330, 340) are mixed together in the dosing chamber (102)

4. The dosing unit (100) of any of claims 2 to 3, wherein the first and second cartridges (304, 306) each comprise a pierceable membrane (308), and wherein, during the first dose setting operation, the first and second needles (116, 118) pierce the membranes (308) of the first and second cartridges (304, 306), respectively.

5. The dosing unit (100) of any of claims 1 to 4, further comprising an injection needle (120) in fluid communication with the dosing chamber (102).

6. The dosing unit (100) of any of claims 1 to 5 further comprising a dose stopper (126) operable to move with respect to the dosing chamber (102).

7. The dosing unit (100) of claim 6 wherein, during an injection step, a displacement of the dose stopper (126) displaces a medicament contained in the dosing chamber (102) in the direction of the injection needle (120).

8. The dosing unit (100) of any of claims 6 to 7, wherein the dosing stopper (126) comprises at least one beveled edge.

9. The dosing unit (100) of any of claims 6 to 8, wherein, during the dose setting step, the transfer of at least one of the first and second medicament (330, 340) from at least one of the first and second reservoirs (304, 306) through at least one of the first and second flow channels (104, 106) to the dosing chamber (102) forces the dosing stopper (126) to move in a proximal direction through the dosing chamber (102).

10. The dosing unit (100) of any of claims 1 to 9, further comprising:
an injection-needle access mechanism (122), wherein while the injection-needle access mechanism (122) resides in a first position access from the dosing chamber (102) to an injection needle (120) is prevented, and while the injection-needle access mechanism (122) resides in a second position access from the dosing chamber (102) to the injection needle (120) is allowed.

11. The dosing unit (100) of claim 10 wherein the injection needle access mechanism (122) comprises a sliding or a rotary injection needle access mechanism.

12. The dosing unit (100) of any of claims 1 to 11 wherein at least one of the first flow channel (104) and the second flow channel (106) comprises a valve system.

13. The dosing unit (100) of claim 12 wherein the valve system comprises a non-return valve that prevents flow from the dosing chamber (102) to a flow channels (104, 106).

14. An injection device comprising:
a dosing chamber (102);
a first cartridge (304) and a second cartridge (306), wherein the first cartridge (304) contains a first medicament (330) and the second cartridge (306) contains a second medicament (340); and
a first flow channel (104) and a second flow channel (106), the first and second flow channels (104, 106) each having a distal end (110, 114) and a proximal end (108, 112), wherein the distal end (110) of the first flow channel (104) is in fluid communication with a first needle (116) in communication with the first reservoir (304) and the proximal end (108) of the first flow channel (104) is connected to the dosing chamber (102),
wherein the distal end (114) of the second flow channel (106) is in fluid communication with a second needle (118) in communication with the second reservoir (306) and the proximal end (112) of the second flow channel (106) is connected to the dosing chamber (102);
an injection needle (120) operable to communicate with the dosing chamber (102); and
a dose stopper (126) mechanically linked to a piston rod (128) and operable to move axially with respect to the dosing chamber (102),
wherein, during a dosing process, at least one of the first and second medicament (330, 340), is transferred from at least one of the first and second cartridges (304, 306) through at least one of the first and second flow channels (104, 106) to the dosing chamber (102), and
wherein, during an injecting process, the piston rod (128) moves in a distal direction and displaces a portion of the medicament in the dosing chamber (102) in a distal direction towards the injection needle (120).

15. The device of claim 14 wherein the injection needle (120) operable to communicate with the dosing chamber (102) comprises a replaceable injection needle.

16. A method for variable dosing of a plurality of medicaments in an injection device, the method comprising:
defining a dosing chamber (102) in an injection device;
operably coupling a first reservoir (304) and a second reservoir (306) to the injection device (300), the first reservoir (304) containing a first medicament (330) and the second reservoir (306) containing a second medicament (340); and
defining a first flow channel (104) and a second flow channel (105),
allowing the first flow channel (104) to be in fluid communication with the first reservoir (304) and the dosing chamber (102),
allowing the second flow channel (106) to be in fluid communication with the second reservoir (306) and the dosing chamber (102);
connecting an injection needle (120) to be in fluid communication with the dosing chamber (102); and
linking a dose stopper (126) to a piston rod (128) that is operable to move axially with respect to the dosing chamber (102).

17. The method of claim 16 further comprising the step of
initiating an injection step where the piston rod (128) is subjected to a forward movement and
displacing a medicament in the dosing chamber (102) in the direction of the injection needle (120).

18. The method of claim 17 wherein the displaced medicament in the dosing chamber (102) comprises a mixed medicament (350) of the first and the second medicament (330, 340).
